Europäisches Patentamt

European Patent Office

Office européen des brevets

(19)

(11) Numéro de publication : **0 427 633 B1**

(12) ## FASCICULE DE BREVET EUROPEEN

(45) Date de publication du fascicule du brevet :
20.10.93 Bulletin 93/42

(51) Int. Cl.⁵ : **A61K 37/66**

(21) Numéro de dépôt : **90403174.7**

(22) Date de dépôt : **08.11.90**

(54) **Utilisation d'un polypeptide ayant l'activité de l'interféron gammma pour la préparation de compositions pharmaceutiques destinées au traitement de cancers primitifs de la plèvre.**

(30) Priorité : **10.11.89 FR 8914780**

(43) Date de publication de la demande :
**15.05.91 Bulletin 91/20**

(45) Mention de la délivrance du brevet :
**20.10.93 Bulletin 93/42**

(84) Etats contractants désignés :
**AT BE CH DE DK FR GB IT LI LU NL SE**

(56) Documents cités :
**WO-A-87/05518**
**WO-A-88/03411**
**PROCEEDINGS OF AMERICAN ASSOCIATION FOR CANCER RESEARCH vol. 27, mars 1986, Los Angeles page 191 COX W et al: "Phase I trial of subcutaneous recombinant DNA gamma interferon"**

(56) Documents cités :
**J. Clin. Oncol. (1988), vol. 6, pp. 689-695**
**AACR (1986), vol. 27, no. 756, Cox et al.**
**Cancer Chemother. Pharmacol. (1987), vol. 19, pp. 233-239**

(73) Titulaire : **ROUSSEL-UCLAF**
**35, Boulevard des Invalides**
**F-75007 Paris (FR)**

(72) Inventeur : **Brandely, Maud**
**135, Boulevard Malesherbes**
**F-75017 Paris (FR)**
Inventeur : **Lando, Danielle**
**17-19, rue de La Plaine**
**F-75020 Paris (FR)**

(74) Mandataire : **Bourgouin, André et al**
**Département des Brevets ROUSSEL UCLAF**
**111, route de Noisy B.P. no 9**
**F-93230 Romainville (FR)**

EP 0 427 633 B1

**Description**

La présente invention concerne l'utilisation d'un polypeptide ayant l'activité de l'interféron gamma pour la préparation de compositions pharmaceutiques destinées à être administrées par voie intrapleurale dans le traitement de cancers primitifs de la plèvre.

L'interféron gamma, outre ses propriétés antivirale et antiproliférative, possède une puissante activité immunomodulatrice qui le distingue des interférons alpha et béta. Il stimule les cellules phagocytaires permettant en particulier la lyse de certaines cellules tumorales. L'étude de la tolérance de l'interféron gamma chez des patients cancéreux au stade terminal n'a pas conduit à des observations de rémissions de ces cancers (Vadhan-Raj, S et al. (1986) J. Clin. Oncol. 4 (2) 137-146 ou Van Der Burg, M et al. (1985) J. Biol. Resp. Mod. 4 264-272), et notamment chez un patient atteint de mésothéliome ayant reçu une administration par voie sous cutanée (W. Cox et al. Proceedings of AACR 27, 191 Mars 1986).

Les cancers primitifs de la plèvre comprennent essentiellement les mésothéliomes diffus et plus rarement les sarcomes pour lesquels les traitements existants de chirurgie, de chimiothérapie ou de radiothérapie seuls ou associés entre eux ne présentent pas d'efficacité reconnue.

Ainsi le mésothéliome malin dont l'association avec l'exposition à l'amiante a été suggérée puis confirmée (Antman, KH N.Eng.J.Med. (1980) 303 200-202) dans 10 à 70 % des cas (Antman, KH et al. Am.J.Med. (1980) 68 356-362) et localisé dans la plèvre, de façon plus fréquente que dans le péritoine (rapport environ 2,5/1), a une évolution mortelle en quelques mois, en l'absence de traitement efficace.

L'efficacité de l'interféron gamma sur diverses cellules cancéreuses fraîches humaines, selon le test dit "human tumor cloning system" décrit par Hamburger et al., a été montrée dans la demande de brevet WO 87/05518, par exemple sur des colonies de cancer de l'ovaire, des poumons, des reins et notamment sur des colonies de cancer pleural.

A la suite de ces observations, des études cliniques ont été conduites, par exemple chez des malades ayant un cancer de l'ovaire. Cependant l'efficacité de l'interféron gamma in vivo n'a pas été observée soit en administration par voie intraveineuse (Welander, C E et al. Am. J Clin. Oncol (1988) 11 (4) 465-469), soit selon un protocole utilisant l'administration par voie intrapéritonéale ((D'Acquisto, R et al. J. Clin. Oncol. (1988) 6 (689-695)).

D'une manière générale, on admet que l'action anticancéreuse de l'interféron gamma nécessite son utilisation en association avec d'autres agents thérapeutiques ((Saito, T et al. Cancer Chemother. Pharmacol. (1989) 19 (233-239). Or la demanderesse vient d'obtenir des résultats montrant que l'interféron gamma présente une activité sur les cancers de plèvre.

L'invention concerne donc l'utilisation d'un polypeptide ayant l'activité de l'interféron gamma humain pour préparer une composition pharmaceutique destinée au traitement de cancers primitifs de la plèvre. On entend par polypeptide ayant l'activité de l'interféron gamma l'interféron humain gamma naturel, l'interféron gamma humain recombinant c'est-à-dire obtenu par la technologie de l'ADN recombinant, par exemple comme cela est décrit par Gray, et al. dans Nature (1982) 295 503-508 ou le brevet EP 77670, des allèles ou des dérivés de ces produits tels que décrits par exemple dans la demande de brevet EP 161504.

Les cancers primitifs de la plèvre que concerne l'invention comprennent des cancers tel que le mésothéliome pleural et le sarcome, généralement caractérisés cliniquement par un épanchement pleural.

L'invention a notamment pour objet l'utilisation caractérisée en ce que le cancer de la plèvre est un mésothéliome.

Le mésothéliome pleural est généralement caractérisé cliniquement par des douleurs de la poitrine ou des difficultés respiratoires associées à un épanchement pleural et son diagnostic le différencie de la pleurésie inflammatoire, du cancer primitif du poumon ou de métastases provenant d'un autre cancer primitif. L'invention décrit l'utilisation d'interféron gamma humain dans un traitement dont l'efficacité est montrée par un taux de réponse d'environ 30 % chez des malades atteints de mésothéliome pleural, ayant éventuellement subi une chirurgie d'exérèse mais pas de traitement antérieur de chimiothérapie ou de radiothérapie.

L'invention a particulièrement pour objet l'utilisation caractérisée en ce que le polypeptide ayant l'activité de l'interféron gamma humain est un interféron gamma recombinant pur. Les compositions pharmaceutiques préparées selon l'invention renferment un interféron gamma humain recombinant, des allèles ou des dérivés de celui-ci, tel que décrit ci-dessus, pour lequel on utilise des techniques de purification connues de l'homme de métier, qui permettent de préparer des produits de haute pureté. L'interféron gamma est notamment celui obtenu à partir d'une souche d'E.coli et comportant 143 aminoacides correspondant à la séquence de l'interféron gamma naturel avec une méthionine N-terminale supplémentaire.

L'activité spécifique des produits utilisés dans l'invention est au moins égale à 1 x $10^7$ U/mg, déterminée selon le test classique par mesure de l'activité antivirale par comparaison avec un étalon NIH sur des cellules humaines Wish infectées par le virus de la stomatite vésiculaire et permet d'administrer des doses efficaces qui sont inférieures à la dose maximum tolérée exprimée en milligramme de produit. L'utilisation selon l'invention fait donc appel à des interférons gamma recombinants possédant un haut degré de pureté.

L'invention a plus particulièrement pour objet l'utilisation caractérisée en ce que l'interféron gamma est administré par voie intrapleurale à la dose de 10 à 50 millions d'unités par injection et tout particulièrement celle caractérisée en ce que l'interféron gamma est administré à la dose de 40 millions d'unités.

L'invention a spécialement pour objet l'utilisation caractérisée en ce que l'interféron gamma est administré de façon répétée au moins deux jours non consécutifs par semaine par voie intrapleurale et plus spécialement celle caractérisée en ce que l'interféron gamma est administré de façon répétée pendant au moins deux mois.

La dose administrée, la fréquence de l'injection et la durée du traitement varient en fonction de l'état du malade.

L'interféron gamma est compris dans une composition pharmaceutique, de préférence lyophilisée en flacons-dose contenant de 0,5 à 2 milligrammes de principe actif et que l'on reconstitue avec de l'eau distillée pour injection. La solution obtenue est éventuellement immédiatement diluée à l'aide d'un soluté, par exemple, le chlorure de sodium à 0,9 %, dans le cas de perfusion intrapleurale.

Selon l'utilisation préférée de l'invention, l'interféron gamma a une activité spécifique de $2 \times 10^7$ U/mg, la dose est de 40 millions d'unités par injection, la fréquence de l'injection est de deux fois par semaine et la durée de l'administration est de 2 mois représentant environ 720 millions d'unités et 36 milligrammes d'interféron gamma administrés au total chez le malade en perfusion par voie intrapleurale. Un traitement de maintenance est ensuite préconisé chez le malade par injection sous cutanée d'interféron gamma, selon les conditions décrites ci-dessus.

Les exemples suivants illustrent l'invention :

Exemple 1 : composition pharmaceutique pour injection.

On a réalisé une préparation pour injection par voie sous cutanée de formule :
interféron gamma     1 mg
excipient             50 mg
eau stérile           1 ml

Exemple 2 : composition pharmaceutique pour injection.

On a réalisé une préparation pour perfusion par voie intrapleurale de formule :
interféron gamma           2 mg
excipient                   100 mg
eau stérile                 10 ml
chlorure de sodium à 0,9 %   100 ml

Exemple 3 : étude clinique dans le traitement de mésothéliome pleural.

L'étude inclue des malades ayant un mésothéliome pleural histologiquement confirmé, confiné à la plèvre, éventuellement étendu au poumon, au thorax ou aux ganglions lymphatiques (stades I, II ou III selon la classification de Butchart) et n'ayant eu aucun traitement antérieur par chimiothérapie ou radiothérapie.

Les malades peuvent avoir éventuellement subi un traitement par chirurgie d'exérèse et présenter une récurrence de la maladie.

Les compositions d'interféron gamma préparée selon l'invention permettent d'injecter des doses de 40 millions d'unités, soit 2 mg par injection, à raison de 2 injections par semaine pendant 2 mois en perfusion de 6 heures par voie intrapleurale. On utilise les compositions décrites à l'exemple 2.

Les malades qui présentent une réponse complète ou partielle sont ensuite, lorsque cela est possible, mis sous traitement de maintenance par voie sous cutanée par injection de dose de 40 millions d'unités, soit 2 milligrammes par injection, à raison de 2 injections par semaine aussi longtemps que possible. On utilise les compositions décrites à l'exemple 1.

Les malades qui présentent une rechute ultérieure ou seulement soit une réponse partielle soit une stabilisation de la maladie sont à nouveau traités par voie intrapleurale comme décrit ci-dessus.

Les lésions tumorales des malades sont évaluées avant et à la fin du traitement par la mesure des lésions macroscopiques au scanner thoracique et à la thorascopie. Une confirmation histologique est obtenue par biopsies multiples des zones précédemment envahies et biopsies au hasard des plèvres pariétale et diaphragmatique.

Sur 13 malades réévalués, les réponses suivantes ont été obtenues :

| PATIENT | SEXE | AGE (ans) | TYPE histologique | STADE | ASPECT ENDOSCOPIQUE | REPONSE |
|---------|------|-----------|-------------------|-------|---------------------|---------|
| HO | H | 66 | Epithelial | 1A | Lymphangite | RC |
| RA | H | 48 | Epithelial | 1A | nodules<5 mm | RC |
| LL | H | 42 | Epithelial | 1A | nodules<5 mm | RC |
| FE | H | 58 | Epithelial | 1A | nodules<5 mm | RC |
| GO | H | 69 | Epithelial | III | | Echec |
| PA | H | 70 | Epithelial | IIA | | Echec |
| TR | H | 65 | Nonévaluable | IIA | | Echec |
| GU | H | 65 | Nonévaluable | IIA | | Echec |
| CH | H | 60 | Nonévaluable | IIA | | Echec |
| FE | H | 54 | Mixte | III | | Echec |
| RA | H | 68 | Epithelial | IIA | | Echec |
| PE | H | 54 | Epithelial | IIA | | Echec |
| THO | H | 41 | Epithelial | IIA | | Echec |

Les résultats montrent 4 réponses complètes (RC), soit un taux de réponse d'environ 30%, comprenant une réponse complète macroscopique mais avec persistance de cellules tumorales à l'examen histologique.

**Revendications**

1.  Utilisation d'un polypeptide ayant l'activité de l'interféron gamma humain pour préparer une composition pharmaceutique destinée à être administrée par voie intrapleurale dans le traitement de cancers primitifs de la plèvre.

2.  Utilisation selon la revendication 1 caractérisée en ce que le cancer de la plèvre est un mésothéliome.

3.  Utilisation selon la revendication 2 caractérisée en ce que le polypeptide ayant l'activité de l'interféron gamma humain est un interféron gamma recombinant pur.

4.  Utilisation selon la revendication 3 caractérisée en ce que l'interféron gamma est administré à la dose de 10 à 50 millions d'unités par injection.

5.  Utilisation selon la revendication 4 caractérisée en ce que l'interféron gamma est administré à la dose de 40 millions d'unités.

6.  Utilisation selon la revendication 5 caractérisée en ce que l'interféron gamma est administré de façon répétée au moins deux jours non consécutifs par semaine.

7.  Utilisation selon la revendication 6 caractérisée en ce que l'interféron gamma est administré de façon répétée pendant au moins deux mois.

**Patentansprüche**

1.  Verwendung eines Polypeptids mit der Aktivität des menschlichen Gamma-Interferons zur Herstellung einer pharmazeutischen Zusammensetzung, die dazu bestimmt ist, bei der Behandlung von primitivem Pleura-Krebs auf interpleuralem Wege verabreicht zu werden.

2. Verwendung gemäß Anspruch 1, dadurch gekennzeichnet, daß der Pleura-Krebs ein Mesotheliom ist.

3. Verwendung gemäß Anspruch 2, dadurch gekennzeichnet, daß das Polypeptid mit der Aktivität des menschlichen Gamma-Interferons ein reines rekombinantes Gamma-Interferon ist.

4. Verwendung gemäß Anspruch 3, dadurch gekennzeichnet, daß das Gamma-Interferon in einer Dosis von 10 bis 50 Millionen Einheiten durch Injektion verabreicht wird.

5. Verwendung gemäß Anspruch 4, dadurch gekennzeichnet, daß das Gamma-Interferon in einer Dosis von 40 Millionen Einheiten verabreicht wird.

6. Verwendung gemäß Anspruch 5, dadurch gekennzeichnet, daß das Gamma-Interferon wiederholt mindestens zwei nicht aufeinanderfolgende Tage pro Woche verabreicht wird.

7. Verwendung gemäß Anspruch 6, dadurch gekennzeichnet, daß das Gamma-Interferon wiederholt während mindestens zwei Monaten verabreicht wird.

## Claims

1. Use of a polypeptide having the activity of human gamma interferon for preparing a pharmaceutical composition intended to be administered by intrapleural route in the treatment of primary cancers of the pleura.

2. Use according to claim 1 characterized in that the cancer of the pleura is a mesothelioma.

3. Use according to claim 2 characterized in that the polypeptide having the activity of human gamma interferon is a pure recombinant gamma interferon.

4. Use according to claim 3 characterized in that the gamma interferon is administered at a dose of 10 to 50 million units per injection.

5. Use according to claim 4 characterized in that the gamma interferon is administered at a dose of 40 million units.

6. Use according to claim 5 characterized in that the gamma interferon is administered in a repeated fashion for at least two non-consecutive days per week.

7. Use according to claim 6 characterized in that the gamma interferon is administered in a repeated fashion for at least two months.